# EUROPÄISCHE PATENTSCHRIFT

(11) **EP 1 062 978 B1**
(45) Veröffentlichungstag und Bekanntmachung des Hinweises auf die Patenterteilung: **11.01.2006**
(21) Anmeldenummer: 00112742.2
(22) Anmeldetag: 16.06.2000
(51) Int. Cl.: A61N 1/37, A61N 1/365

(54) **Vorrichtung zur Steuerung der Stimulationsamplitude eines Herzschrittmachers**
Device for controlling the stimulation amplitude of a cardiac pacemaker
Appareil de commande de l'amplitude de stimulation d'un stimulateur cardiaque

(30) Priorität: 24.06.1999 DE 19929091
(43) Veröffentlichungstag der Anmeldung: 27.12.2000
(73) Patentinhaber: Biotronik GmbH & Co. KG, 12359 Berlin (DE)
(72) Erfinder: Thong, Tran, Portland, OR 97229 (US)
(74) Vertreter: Lindner-Vogt, Karin L.

(56) Entgegenhaltungen:
- EP-A- 0 850 662
- EP-A- 0 990 451
- US-A- 5 766 230
- US-A- 6 070 100

## Beschreibung

Die Erfindung betrifft ein kardiologisches Implantat, das eine rechts- und linksseitige Stimulation des Herzens unter Verwendung einer linksseitigen Stimulationselektrode in einer Koronarvene und einer Gegenelektrode in der zugeordneten rechten Herzkammer durchführt und insbesondere ein im Steuerprogramm des Implantats implementiertes Verfahren zur Steuerung der Stimulationsamplitude.

Zum Hintergrund der Erfindung ist festzuhalten, daß sich bei kardiologischen Implantaten; wie z.B. Herzschrittmachern oder Defibrillatoren, das Problem einer möglichst lang ausreichenden Energieversorgung stellt. Die Lebensdauer eines Implantates kann dabei einerseits durch Erhöhung der Batteriekapazität, andererseits durch die Verringerung des Energieverbrauches verlängert werden. Die Erfindung setzt sich letzteres zum Ziel.

Der Energieverbrauch eines kardiologischen Implantates wird nun u.a. durch die variabel zu steuernden Amplituden der Reizimpulse bestimmt. Je niedriger die Amplitude eingestellt wird, desto geringer ist der Energieverbrauch des Implantates.

Problematisch dabei ist, daß die Amplitude natürlich nicht beliebig niedrig eingestellt werden kann, da für diesen Fall die Reizschwelle des von dem Implantat unterstützten Organs unterschritten und damit kein Stimulationserfolg mehr erreicht wird. Erschwerend ist in diesem Zusammenhang die Tatsache, daß die Reizschwellen, ab denen eine erfolgreiche Stimulation des Herzens stattfinden kann, zum einen von Patient zu Patient sehr unterschiedlich sein können. Zum anderen ändert sich die Reizschwelle, ab der ein Stimulationsimpuls erfolgreich in eine Herzkontraktion umgesetzt wird, bei ein und demselben Patienten aufgrund physiologischer Änderungen ebenfalls über die Zeit. Dabei sind Zeitkonstanten der Änderungen von wenigen Stunden bis Monaten durchaus gängig.

Moderne Herzschrittmacher haben aus dem vorstehenden Grund eine automatische Steuerung der Stimulationsamplitude, die durch geeignete Detektionsprozesse und Auswerte-Algorithmen die Stimulationsamplitude einerseits unter energetischen Gesichtspunkten möglichst niedrig, andererseits für eine zuverlässige Patientenversorgung auf einem bestimmten Mindestwert hält.

So ist es aus der US-A 5,766,230 bekannt, in prozeß- und schaltungstechnisch sehr aufwendiger Weise während jeder Abgabe eines Stimulationsimpulses zeitaufgelöst den Impedanzverlauf zu verfolgen. Ein erfolgreicher Stimulationsimpuls, der zu einer Kontraktion des Herzens führt, äußert sich im entsprechenden Impedanz-Meßdiagramm durch einen plötzlichen Abfall oder einen Spitzenwert je nach den physiologischen und meßtechnischen Gegebenheiten. Da die Impulserfassung praktisch in Echtzeit durchgeführt wird, kann bereits während eines Impulses ermittelt werden, ob der Impuls zu einer erfolgreichen Stimulation geführt hat. Wird eine solche Stimulation nicht erkannt, kann ein sogenannter "Sicherheitsimpuls" sofort verabreicht werden, der die gewünschte Herzkontraktion bewirkt.

Die vorstehende Detektionsweise bringt einen hohen gerätetechnischen Aufwand mit sich, der insbesondere im Hinblick auf einen möglichst geringen Energieverbrauch der Schaltung von Nachteil ist. Zum einen ist die meßtechnisch aufwendige Echtzeit-Verfolgung der Stimulationsimpedanz nämlich energieaufwendig, zum anderen geht aus der genannten Patentschrift explizit hervor, daß die Stimulationsamplitude um einen hohen Sicherheits-Differenzwert über der eigentlich festgestellten Stimulationsschwelle eingestellt wird. So zeigt ein Zahlenbeispiel in der US-A 5,766,230, daß bei einer sich einstellenden gemessenen Stimulationsschwelle von ca. 1,5 V die eigentliche Stimulationsspannung bei ca. 2,8 V - also fast dem doppelten Wert - liegt. Es wird im Stand der Technik also ein erhebliches Potential zur Energieeinsparung und damit Verlängerung der Lebensdauer kardiologischer Implantate vernachlässigt.

Davon ausgehend liegt der Erfindung die Aufgabe zugrunde, ein kardiologisches Implantat mit einem im Steuerprogramm des Implantats implementierten Verfahren zur Steuerung der Stimulationsamplitude anzugeben, mit dem unter verringertem Energieeinsatz eine zuverlässige und therapiegerechte Stimulation des vom Implantat unterstützten Organs erreicht wird.

Diese Aufgabe wird durch ein Implantat mit einem Steuerprogramm gelöst, in dem ein Steuerungsverfahren mit folgenden Verfahrensschritten implementiert ist:
- Erfassen der Stimulationsimpedanz während der Abgabe linksseitiger Stimulationsimpulse in Form eines für den jeweiligen Stimulationsimpuls repräsentativen Meßwerts als Kriterium für eine erfolgreiche Abgabe eines Stimulationsimpulses,
- Ermitteln und Speichern eines für die Stimulation repräsentativen Impedanz-Vergleichswertes der Stimulationsimpedanz,
- Detektion einer Amplitudenschwelle für eine erfolgreiche Abgabe eines Stimulationsimpulses durch Feststellen einer signifikanten Änderung der erfaßten Stimulationsimpedanz gegenüber dem gespeicherten Vergleichswert der Stimulationsimpedanz, und
- Einstellen der Stimulationsamplitude auf der Basis der detektierten Amplitudenschwelle.

Die Erfindung geht dabei davon aus, daß das vom Implantat zu unterstützende Herz (auch) linksseitig stimuliert wird. Dabei wird sich die Tatsache zunutze gemacht, daß für eine Stimulation des linken Herzens höhere Amplitudenschwellen notwendig sind, als bei einer Stimulation des rechten Herzens, wie es z.B. bei dem Herzschrittmacher gemäß der US-A 5,766,230 eingesetzt wird. Die linksseitige Herzstimulation erfolgt z.B. über eine Ring-Elektrode, die in den Koronarsinus eingeschoben und nahe des linken Atriums oder - falls die Elektrode noch weiter eingeschoben wird - nahe des linken Ventrikels positioniert wird. Stimulation und Wahrnehmung können dann in an sich bekannter Weise entweder unipolar zwischen dieser Elektrode und dem Gehäuse des Implantates oder bipolar zwischen dieser Elektrode und einer weiteren, z.B. im rechten Atrium positionierten Elektrode, vorgenommen werden.

Bei der linksseitigen Stimulation des Herzens wird nun - da die rechtsseitige Stimulation mit niedrigeren Reizschwellen vonstatten geht - eine rechtsseitige Stimulation des Herzens gleichzeitig initiiert. Mit Erreichen der grenzwertigen Stimulationsamplitude für eine linksseitige Stimulation erfolgt also eine rechts- und linksseitige Stimulation des Herzens, wobei ein Stimulationserfolg in der linken Herzseite nicht gewährleistet ist, da nahe unterhalb der Amplitudenschwelle gearbeitet wird. Dies ist allerdings dahingehend unproblematisch, als eine Stimulation nur der rechten Herzseite hämodynamisch unbedenklich ist. Die linke Herzseite kontrahiert nämlich aufgrund der natürlichen Erregungsausbreitung nach einer rechtsseitigen Stimulation von selbst, wenn auch etwas später als bei gleichzeitiger erfolgreicher Stimulation. Diese natürliche Erregungsausbreitung ist auch der Grund dafür, daß herkömmliche Schrittmacher üblicherweise ausschließlich in der rechten Herzseite stimulieren können.

Da nun beim anspruchsgemäßen Verfahren der Stimulationserfolg in der linken Herzseite als Maß für die Einstellung der Stimulationsamplitude herangezogen wird, wird ein natürlicher Abstand zur absoluten Reizschwelle der rechten Herzseite ermittelt, unterhalb derer gar kein Stimulationserfolg mehr eintritt. Dies ist der Abstand, der beim Stand der Technik durch Verwenden einer Sicherheits-Marge gewährleistet werden soll. Diese Sicherheits-Marge ist beim Stand der Technik mehr oder weniger willkürlich aufgrund von Erfahrungen gewählt, während beim anspruchsgemäßen Verfahren ein echter physiologischer Parameter, nämlich der linksseitige Stimulationserfolg, herangezogen wird.

Der für die Stimulation repräsentative Impedanz-Vergleichswert der Stimulationsimpedanz kann dabei als Referenzwert bei der Inbetriebnahme des kardiologischen Implantates ermittelt, angegeben und gespeichert werden. Vorzugsweise wird der Impedanz-Vergleichswert jedoch laufend als Einzel- oder Durchschnittswert der Impedanz über mehrere Stimulationsimpulse hinweg ermittelt und aktualisiert abgespeichert.

Die erfindungsgemäß vorgesehene Detektion der Amplitudenschwelle kann dabei durch einen laufenden Vergleich mit dem Impedanz-Vergleichswert auf der Basis einer signifikanten Verringerung der Stimulationsimpedanz gegenüber dem Impedanz-Vergleichswert bei einer nichterfolgreichen Abgabe eines linksseitigen Stimulationsimpulses erfolgen. Dies bedeutet, daß bei stetig erfolgreicher Stimulierung immer mit einer gleichbleibenden Stimulationsamplitude gearbeitet wird.

Alternierend hierzu kann die Amplitudenschwelle durch sukzessives Erhöhen der Amplitude der Stimulationsimpulse praktisch abgescannt werden, bis eine erfolgreiche Herzstimulation linksseitig durch Feststellen einer signifikanten Erhöhung der Stimulationsimpedanz gegenüber dem Vergleichs-Impedanzwert detektiert wird.

Falls hinsichtlich besonderer Therapie-Formen gewünscht, kann die Stimulationsamplitude zwar um einen Puffer-Differenzwert über der detektierten Amplitudenschwelle eingestellt werden. Diese Sicherheits-Marge kann jedoch aufgrund der Heranziehung des linksseitigen Stimulationserfolg als Basis für die Einstellung der Amplitudenschwelle weitaus geringer ausfallen, als die Sicherheits-Marge beim Stand der Technik, wo ein Faktor 2 eingesetzt wird.

In einer bevorzugten Weiterbildung der Erfindung kann bei Ausbleiben des Stimulationserfolges auf der linken Herzseite eine zuverlässige Verfahrensroutine eingesetzt werden, um die Amplitudenschwelle den offensichtlich geänderten Verhältnissen anzupassen. Demnach wird die Stimulationsamplitude auf einen Maximalwert gesetzt und dann sukzessive während der folgenden Stimulationszyklen erniedrigt, bis wieder ein Ausbleiben des Stimulationserfolges festgestellt wird. Dieser Zustand definiert die neue Amplitudenschwelle, bei der zwar aufgrund der Grenzwertigkeit zur linksseitigen Stimulierung auf dieser Herzseite ein Erfolg nicht unbedingt gewährleistet ist, jedoch wird ein ausreichender, physiologisch definierter Abstand zur Reizschwelle der rechten Herzseite eingehalten, was insoweit zu einer zuverlässigen Arbeitsweise des Implantates führt.

Zur Erhöhung der Zuverlässigkeit kann gemäß einer bevorzugten Weiterbildung der Erfindung nach Feststellen eines Ausbleibens des Stimulationserfolges die Stimulationsamplitude für mindestens einen Impuls auf einen Maximalwert gesetzt werden.

Eine besonders bevorzugte Weiterbildung der Erfindung ist für eine tripolare Stimulationsanordnung mit einer linksseitigen Stimulationselektrode in einer Koronarvene, einer rechtsseitigen Stimulationselektrode in einer zugeordneten rechten Herzkammer und einer Gegenelektrode angegeben. Demnach kann bei der Detektion der Amplitudenschwelle die Stimulationsimpedanz auf eine signifikante Impedanzerhöhung beim Übergang von einer nur rechtsseitig erfolgenden Stimulation auf eine beidseitig erfolgende Stimulation überwacht und die Stimulations-amplitude auf den beim Übergang festgestellten Amplitudenwert eingestellt werden. Bei dieser Auslegung des erfindungsgemäßen Verfahrens werden die Möglichkeiten einer tripolaren Elektrodenanordnung optimal ausgenutzt. Aufgrund der durch die tripolare Anordnung möglich gewordenen meßtechnischen Differenzierung zwischen ausschließlich rechtsseitiger Stimulation und beidseitiger Stimulation ist es unbedenklich, bei Feststellen eines linksseitig ausgebliebenen Stimulationserfolges den Amplitudenwert der Amplitudenschwelle nicht zu erhöhen. Damit wird die eingangs erörterte Aufgabenstellung eines möglichst geringen Energieeinsatzes in besonderer Weise verfolgt.

Entgegen dem diskutierten Stand der Technik ist es bei der Erfindung möglich, den für den jeweiligen Stimulationsimpuls repräsentativen Meßwert der Stimulationsimpedanz durch kontinuierliches Aufzeichnen und Auswerten von Strom und Spannung als über den Impuls integrierter oder gemittelter Durchschnittswert zu erfassen. Bei der Implementierung des anspruchsgemäßen Verfahrens ist also keine aufwendige Echtzeit-Überwachung des Impulses notwendig, es muß lediglich ein Durchschnittswert verarbeitet werden.

Dieser Wert kann in nochmaliger Vereinfachung auch dadurch ermittelt werden, daß lediglich zu einem definierten Zeitpunkt nach Beginn des Impulses der Stimulationsstrom gemessen und zu der geräteseitig eingestellten Stimulationsspannung zur Berechnung der Impedanz in Beziehung gesetzt wird.

Weitere Merkmale, Einzelheiten und Vorteile der Erfindung ergeben sich aus der nachfolgenden Beschreibung eines Ausführungsbeispieles anhand der beigefügten Zeichnung.

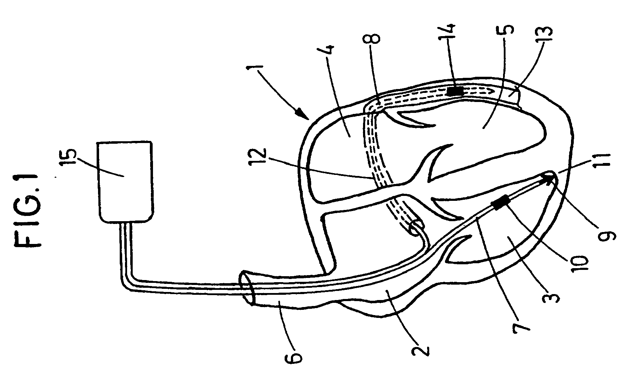

- Fig. 1: zeigt eines schematisches Schaubild eines herzschrittmachergestützten Herzens mit einer tripolaren Elektrodenanordnung.

In der Zeichnung ist mit 1 als schematischer Schnitt ein Herz mit seinem rechten Atrium 2, rechten Ventrikel 3, linken Atrium 4 und linken Ventrikel 5 gezeigt. Über die Vena cava 6 sind zwei Katheter 7, 8 in das Herz eingeführt, von denen der eine Katheter 7 über das rechte Atrium 2 in das rechte Ventrikel 3 vorgeschoben ist. An diesem Katheter 7 sind eine Spitzenelektrode 9 und eine Ringelektrode 10 angebracht. Die Spitzenelektrode 9 ist an der Spitze des rechten Ventrikels 3 im Myokard 11 verankert. Die Ringelektrode 10 floatet frei im Blutstrom innerhalb des rechten Ventrikels 3.

Der zweite Katheter 8 ist über den Koronarsinus 12 bis zur sich daran anschließenden großen Herzvene 13 vorgeschoben, womit eine weitere Ringelektrode 14 in dieser großen Herzvene 13 positioniert wird.

Die beiden Katheter 7, 8 führen zu einem üblicherweise subkutan implantierten Herzschrittmacher 15, an den die Elektroden 9, 10, 14 über entsprechende Eingänge angeschlossen sind. Der apparative Aufbau des Herzschrittmachers 15 ist üblich, wozu z.B. neben einer geeigneten Energieversorgung auf der Basis einer Batterie und einer Telemetrie-Datenübertragungseinrichtung zur Programmierung des Herzschrittmachers und zum Abrufen von Daten jeweils von außen auch eine Mikroprozessor-gestützte zentrale Steuereinheit gehört, in der ein entsprechendes Steuerprogramm implementiert ist. Dieses setzt bestimmte Routinen und Verfahren zur Abgabe von Stimulationsimpulsen mit bestimmten Pulsformen, Stimulationsamplituden, zeitlichen Abfolgen, usw. um, wie es für die Therapie des in seiner Funktion gestörten Herzens 1 notwendig ist.

In dieser Hinsicht ist auch ein Verfahren zur Steuerung der Stimulationsamplitude des Herzschrittmachers 15 gemäß der Erfindung in dem Steuerprogramm des Schrittmachers implementiert. Ausgegangen wird dabei von einer tripolaren Anordnung mit der Spitzenelektrode 9 und Ringelektrode 14 als Kathoden und der Ringelektrode 10 als anodische Gegenelektrode. Es findet also eine links- und rechtsseitige ventrikuläre - also "biventrikuläre" - Stimulierung des Herzens durch Abgabe entsprechender Stimulationsimpulse statt. Diese Stimulationsimpulse sind auf eine bestimmte Stimulationsamplitude einzustellen, wofür ein bestimmter Amplitudenschwellenwert ermittelt werden muß, der eine zuverlässige Stimulierung des Herzens gewährleistet. Dazu wird die Stimulationsimpedanz während der Abgabe von Stimulationsimpulsen über die Elektroden 9, 14, 10 erfaßt, indem der über die Spitzenelektrode 9 und Ring-elektrode 14 verabreichte Stimulationsstrom zu einem definierten Zeitpunkt, z.B. 30 µsec nach Beginn des bis zu zwei msec dauernden Stimulationsimpulses gemessen und mit der geräteseitig eingestellten Stimulationsspannung zur Berechnung der Impedanz in Beziehung gesetzt wird. Hierbei wurde durch medizinische Meßreihen herausgefunden, daß bei einem nur rechtsseitigen Stimulationserfolg die Stimulationsimpedanz z.B. 230 Ohm betrug, wogegen bei einer beidseitigen Stimulation dieser Wert bei 290 Ohm lag. Ein entsprechender Impedanz-Vergleichswert kann in einem entsprechenden Speicher in der Mikroprozessorsteuerung des Herzschrittmachers abgespeichert werden. Beim Betrieb des Herzschrittmachers wird dann die erfaßte Stimulationsimpedanz mit dem gespeicherten Vergleichswert verglichen und bei einer signifikanten Änderung während eines Stimulationsimpulses die dabei eingesetzte Stimulationsamplitude als Amplitudenschwelle für eine erfolgreiche Abgabe eines Stimulationsimpulses detektiert. Entsprechend wird dann die Stimulationsamplitude eingestellt.

Im laufenden Betrieb kann der Impedanz-Vergleichswert als Durchschnittswert über mehrere Stimulationsimpulse hinweg ermittelt und jeweils aktualisiert abgespeichert werden. Damit findet quasi eine passive Detektion der Ampitudenschwelle statt, da während des laufenden Betriebes gemessen wird.

Eine aktive Detektion kann auf der Basis der vorstehend erwähnten Erhöhung der Stimulationsimpedanz bei nur rechtsseitigem bzw. rechts- und linksseitigem Stimulationserfolg durchgeführt werden, in dem eine Amplitudenschwelle ermittelt wird durch sukzessives Erhöhen der Amplitude der Stimulationsimpulse, bis eine erfolgreiche Herzstimulation durch Feststellen einer signifikanten Erhöhung der Stimulationsimpedanz detektiert wird.

Dabei wird speziell der Übergang von einem niedrigeren Impedanzwert bei nur rechtsseitig erfolgender Stimulation auf den höheren Impendanzwert für eine beidseitig erfolgende Stimulation überwacht.

Anschließend wird die Stimulationsamplitude auf den beim Übergang festgestellten Amplitudenwert eingestellt, so daß der Herzschrittmacher praktisch immer an der Grenze zur beidseitigen Stimulation arbeitet. Aufgrund von Schwankungen der notwendigen Reizschwellen kann es nun zwar geschehen, daß die Stimulationsamplitude für eine beidseitige Stimulation zu gering ist, da sie jedoch ein ausreichendes Maß über dem für die rechtsseitige Stimulation notwendigen Niveau liegt, ist diese rechtsseitig Stimulation praktisch jederzeit gewährleistet. Insoweit kann bei einem linksseitig ausgebliebenen Stimulationserfolg auch eine Erhöhung des Amplitudenwertes ausbleiben.

Falls nur eine rechtsseitige oder gar keine Stimulation festgestellt wurde, kann die Stimulationsamplitude für den nachfolgenden Impuls auch auf einen Maximalwert gesetzt und anschließend bei weiteren folgenden Impulsen sukzessive erniedrigt werden. Es wird also ein Zustand eingestellt, bei dem auf hohem Energieniveau, also beidseitig stimuliert wird. Die Grenze zwischen beidseitiger und ausschließlich rechtsseitiger Stimulierung wird durch die erwähnte Überwachung des Impedanzwertes festgestellt, wonach die Amplitudenschwelle auf diesen Grenzwert eingestellt wird. Damit erfolgt eine Anpassung an die neue Amplitudenschwelle.

## Patentansprüche

1. Kardiologisches Implantat, das eine rechts- und linksseitige Stimulation des Herzens unter Verwendung einer linksseitigen Stimulationselektrode (14) in einer Koronarvene (13) durchführt, **gekennzeichnet durch** ein im Steuerprogramm des Implantats implementiertes Verfahren zur Steuerung der Stimulationsamplitude mit folgenden Verfahrensschritten:
- Erfassen der Stimulationsimpedanz während der Abgabe linksseitiger Stimulationsimpulse in Form eines für den jeweiligen Stimulationsimpuls repräsentativen Meßwerts als Kriterium für eine erfolgreiche Abgabe eines Stimulationsimpulses,
- Ermitteln und Speichern eines für die Stimulation repräsentativen Impedanz-Vergleichswertes der Stimulationsimpedanz,
- Detektion einer Amplitudenschwelle für eine erfolgreiche Abgabe eines Stimulationsimpulses **durch** Feststellen einer signifikanten Änderung der erfaßten Stimulationsimpedanz gegenüber dem gespeicherten Vergleichswert der Stimulationsimpedanz, und
- Einstellen der Stimulationsamplitude auf der Basis der detektierten Amplitudenschwelle.

2. Implantat nach Anspruch 1, **dadurch gekennzeichnet, daß** der Impedanz-Vergleichswert laufend als Einzel- oder Durchschnittswert über mehrere Stimulationsimpulse ermittelt und aktualisiert abgespeichert wird.

3. Implantat nach Anspruch 1 oder 2, **gekennzeichnet durch** eine Detektion der Amplitudenschwelle auf der Basis einer signifikanten Verringerung der Stimulationsimpedanz gegenüber dem Impedanz-Vergleichswert aufgrund einer nichterfolgreichen Abgabe eines Stimulationsimpulses.

4. Implantat nach einem der Ansprüche 1 bis 3, **gekennzeichnet durch** eine aktive Detektion der Amplitudenschwelle **durch** sukzessives Erhöhen der Amplitude der Stimulationsimpulse, bis eine erfolgreiche Herzstimulation **durch** Feststellen einer signifikanten Erhöhung der Stimulationsimpedanz gegenüber dem Impedanz-Vergleichswert detektiert wird.

5. Implantat nach Anspruch 4, **gekennzeichnet durch** regelmäßiges Wiederholen der Amplitudenschwellen-Detektion zur laufenden Aktualisierung der Amplitudenschwelle.

6. Implantat nach einem der Ansprüche 1 bis 5, **dadurch gekennzeichnet, daß** die Stimulationsamplitude um einen Puffer-Differenzwert über der Amplitudenschwelle eingestellt wird.

7. Implantat nach einem der Ansprüche 1 bis 6, **dadurch gekennzeichnet, daß** nach Feststellen einer nicht-erfolgreichen Abgabe eines Stimulationsimpulses die Stimulationsamplitude für einen nachfolgenden Impuls auf einen Maximalwert gesetzt und anschließend die Amplitude sukzessive erniedrigt wird, bis ein Ausbleiben des Stimulatonserfolges die Amplitudenschwelle definiert.

8. Implantat nach einem der Ansprüche 1 bis 7, **dadurch gekennzeichnet, daß** nach Feststellen eines Ausbleibens des Stimulationserfolges die Stimulationsamplitude für mindestens einen Impuls auf einen Maximalwert gesetzt wird.

9. Implantat nach einem der Ansprüche 1 bis 8 unter Verwendung einer tripolaren Stimulationsanordnung mit einer linksseitigen Stimulationselektrode (14) in einer Koronarvene (13), einer rechtsseitigen Stimulationselektrode (9) in einer zugeordneten rechten Herzkammer (3) und einer Gegenelektrode (10), **dadurch gekennzeichnet, daß** bei der Detektion der Amplitudenschwelle die Stimulationsimpedanz auf eine signifikante Impedanzerhöhung beim Übergang von einer nur rechtsseitig erfolgenden Stimulation auf eine beidseitig erfolgende Stimulation überwacht und die Stimulationsamplitude auf den beim Übergang festgestellten Amplitudenwert eingestellt wird.

10. Implantat nach Anspruch 9, **dadurch gekennzeichnet, daß** bei Feststellen eines linksseitig ausgebliebenen Stimulationserfolges der Amplitudenwert nicht erhöht wird.

11. Implantat nach einem der Ansprüche 1 bis 10, **dadurch gekennzeichnet, daß** als für den jeweiligen Stimulationsimpuls repräsentativer Impedanz-Meßwert ein durch kontinuierliches Aufzeichnen und Auswerten von Strom und Spannung über den Impuls integrierter oder gemittelter Durchschnittswert der Impedanz erfaßt wird.

12. Implantat nach einem der Ansprüche 1 bis 10, **dadurch gekennzeichnet, daß** zur Bestimmung des für den jeweiligen Stimulationsimpuls repräsentativen Impedanz-Meßwerts zu einem definierten Zeitpunkt nach Beginn des Impulses der Stimulationsstrom gemessen und mit der geräteseitig eingestellten Stimulationsspannung zur Berechnung der Impedanz in Beziehung gesetzt wird.

## Claims

1. A cardiological implant, which performs right-side and left-side stimulation of the heart using a left-side stimulation electrode (14) in a coronary vein (13), **characterized by** a method for controlling the stimulation amplitude, the method being implemented in the control program of the implant and having the following method steps:
- detecting the stimulation impedance during the delivery of left-side stimulation pulses in the form of a measured value representative of the particular stimulation pulse as a criterion for a successful delivery of a stimulation pulse,
- determining and storing an impedance comparative value of the stimulation impedance representative for the stimulation,
- detecting an amplitude threshold for a successful delivery of the stimulation pulse by establishing a significant change of the detected stimulation impedance in relation to the stored comparative value of the stimulation impedance, and
- setting the stimulation amplitude on the basis of the detected amplitude threshold.

2. The implant according to claim 1, **characterized in that** the impedance comparative value is determined running as an individual or average value over multiple stimulation pulses and is stored in updated form.

3. The implant according to claim 1 or 2, **characterized by** a detection of amplitude threshold on the basis of a significant reduction of the stimulation impedance in relation to the impedance comparative value because of an unsuccessful delivery of a stimulation pulse.

4. The implant according to one of claims 1 through 3, **characterized by** an active detection of amplitude threshold through successive increase of the amplitude of the stimulation pulses until a successful heart stimulation is detected by establishing a significant increase of the stimulation impedance in relation to the impedance comparative value.

5. The implant according to claim 4, **characterized by** regular repetition of the amplitude threshold detection for the running update of the amplitude threshold.

6. The implant according to one of claims 1 through 5, **characterized in that** the stimulation amplitude is set above the amplitude threshold by a buffer differential value.

7. The implant according to one of claims 1 through 6, **characterized in that** after establishing an unsuccessful delivery of the stimulation pulse, the stimulation amplitude for a following pulse is set to a maximum value and subsequently the amplitude is successively reduced until absence of stimulation result defines the amplitude threshold.

8. The implant according to one of claims 1 through 7, **characterized in that** after establishing an absence of the stimulation result, the stimulation amplitude is set to a maximum value for at least one pulse.

9. The implant according to one of claims 1 through 8 using a tripolar stimulation system having a left-side stimulation electrode (14) in a coronary vein (13), a right-side stimulation electrode (9) in an assigned right cardiac chamber (3), and a counter electrode (10), **characterized in that** upon the detection of the amplitude threshold, the stimulation impedance is monitored for a significant impedance increase upon transition from a stimulation only performed on the right side to a stimulation performed on both sides and the stimulation amplitude is set to the amplitude value established upon the transition.

10. The implant according to claim 9, **characterized in that**, if the absence of a stimulation result is established on the left side, the amplitude value is not increased.

11. The implant according to one of claim 1 through 10, **characterized in that** an average value of the impedance, which is integrated or averaged through continuous recording and analysis of current and voltage via the pulse, is detected as the impedance measured value representative for the particular stimulation pulse.

12. The implant according to one of claims 1 through 10, **characterized in that**, to determine the impedance measured value representative for the particular stimulation pulse, the stimulation current is measured at a defined instant after beginning the pulse and is related to the stimulation voltage set in the device to calculate the impedance.

## Revendications

1. Implant cardiologique, qui effectue une stimulation côté droit et côté gauche du coeur avec l'utilisation d'une électrode de stimulation (14) côté gauche dans une veine coronarienne (13), **caractérisé par** un procédé implanté dans le programme de commande de l'implant pour la commande de l'amplitude de stimulation avec les étapes de procédé suivantes :
- enregistrement de l'impédance de stimulation pendant l'envoi d'impulsions de stimulation côté gauche sous la forme d'une valeur de mesure représentative de l'impulsion de stimulation concernée comme critère d'un envoi réussi d'une impulsion de stimulation,
- détermination et mémorisation d'une valeur de comparaison d'impédance, représentative de la stimulation, de l'impédance de stimulation,
- détection d'un seuil d'amplitude pour un envoi réussi d'une impulsion de stimulation par constatation d'une modification significative de l'impédance de stimulation enregistrée par rapport à la valeur de comparaison mémorisée de l'impédance de stimulation, et
- réglage de l'amplitude de stimulation sur la base du seuil d'amplitude détecté.

2. Implant selon la revendication 1, **caractérisé en ce que** la valeur de comparaison d'impédance est déterminée en permanence en tant que valeur individuelle ou valeur moyenne au moyen de plusieurs impulsions de stimulation et est mémorisée sous une forme actualisée.

3. Implant selon la revendication 1 ou 2, **caractérisé par** une détection du seuil d'amplitude sur la base d'une réduction significative de l'impédance de stimulation par rapport à la valeur de comparaison d'impédance sur la base d'un envoi non réussi d'une impulsion de stimulation.

4. Implant selon l'une quelconque des revendications 1 à 3, **caractérisé par** une détection active du seuil d'amplitude par une élévation successive de l'amplitude des impulsions de stimulation, jusqu'à ce qu'une stimulation cardiaque réussie soit détectée par la constatation d'une élévation significative de l'impédance de stimulation par rapport à la valeur de comparaison d'impédance.

5. Implant selon la revendication 4, **caractérisé par** une répétition régulière de la détection du seuil d'amplitude pour l'actualisation permanente du seuil d'amplitude.

6. Implant selon l'une quelconque des revendications 1 à 5, **caractérisé en ce que** l'amplitude de stimulation est réglée autour d'une valeur de différence tampon au-dessus du seuil d'amplitude.

7. Implant selon l'une quelconque des revendications 1 à 6, **caractérisé en ce que**, après la constatation d'une émission non réussie d'une impulsion de stimulation, l'amplitude de stimulation pour une impulsion consécutive est placée sur une valeur maximale et ensuite l'amplitude est abaissée de façon successive jusqu'à ce qu'un échec d'un essai de stimulation définisse le seuil d'amplitude.

8. Implant selon l'une quelconque des revendications 1 à 7, **caractérisé en ce que**, après la constatation d'un échec de l'essai de stimulation, l'amplitude de stimulation est placée sur une valeur maximale pour au moins une impulsion.

9. Implant selon l'une quelconque des revendications 1 à 8, avec l'utilisation d'un dispositif de stimulation tripolaire avec une électrode de stimulation (14) côté gauche dans une veine coronarienne (13), une électrode de stimulation (9) côté droit dans un ventricule (3) attribué et une contre-électrode (6), **caractérisé en ce que**, lors de la détection du seuil d'amplitude, l'impédance de stimulation est contrôlée au niveau d'une élévation d'impédance significative lors du passage d'une stimulation effectuée seulement côté droit à une stimulation effectuée des deux côtés et l'amplitude de stimulation est réglée sur la valeur d'amplitude constatée lors de ce passage.

10. Implant selon la revendication 9, **caractérisé en ce que**, en cas de constatation d'un essai de stimulation raté côté gauche, la valeur d'amplitude n'est pas augmentée.

11. Implant selon l'une quelconque des revendications 1 à 10, **caractérisé en ce que**, comme valeur de mesure d'impédance représentative de l'impulsion de stimulation respective, on enregistre une valeur moyenne de l'impédance qui est intégrée et calculée par l'enregistrement et l'analyse continus du courant et de la tension.

12. Implant selon l'une quelconque des revendications 1 à 10, **caractérisé en ce que**, pour la détermination de la valeur de mesure d'impédance représentative de l'impulsion de stimulation respective, on mesure le courant de stimulation à un moment défini après le début de l'impulsion et on la met en rapport avec la tension de stimulation réglée côté appareil pour le calcul de l'impédance.
